# EUROPÄISCHE PATENTSCHRIFT

(11) **EP 3 441 452 B1**
(45) Veröffentlichungstag und Bekanntmachung des Hinweises auf die Patenterteilung: **05.02.2020**
(21) Anmeldenummer: 18000536.5
(22) Anmeldetag: 19.06.2018
(51) Int. Cl.: C12M 1/107, C12M 1/16, C12M 1/00, C10L 3/08, C25B 1/04, F01K 13/00

(54) **ENERGIEERZEUGUNGSSYSTEM MIT EINEM HEIZKRAFTWERK UND EINER VERGÄRUNGSANLAGE UND VERFAHREN ZUR ENERGIEERZEUGUNG**
ENERGY GENERATING SYSTEM INCLUDING COGENERATION PLANT AND A FERMENTATION PLANT AND METHOD FOR THE PRODUCTION OF ENERGY
SYSTÈME DE PRODUCTION D'ÉNERGIE DOTÉ D'UNE CENTRALE COMBINÉE ET D'UNE INSTALLATION DE FERMENTATION ET PROCÉDÉ DE PRODUCTION D'ÉNERGIE

(30) Priorität: 10.08.2017 DE 102017007547
(43) Veröffentlichungstag der Anmeldung: 13.02.2019
(73) Patentinhaber: Martin GmbH für Umwelt- und Energietechnik, 80807 München (DE)
(72) Erfinder: MARTIN, Ulrich, 81247 München (DE); SCHÖNSTEINER, Josef, 93073 Neutraubling (DE); VON RAVEN, Robert, 82402 Seeshaupt (DE)
(74) Vertreter: Castell, Klaus

(56) Entgegenhaltungen:
- EP-A1- 1 634 946
- WO-A1-2015/120983
- DE-A1-102009 018 126
- DE-C1- 19 621 751

## Beschreibung

Die Erfindung betrifft ein Energieerzeugungssystem mit einem Heizkraftwerk einer Vergärungsanlage und einer Elektrolyseanlage, die über eine Leitung mit der Vergärungsanlage in Verbindung steht.

Ein derartiges System ist aus der WO 2015/120983 A1 bekannt. Bei dieser Anlage wird in einem exothermen Prozess aus Wasserstoff und Kohlendioxid Methangas und Wasser erzeugt. Außerdem ist aus der EP 1 634 946 A1 ein System bekannt, bei dem Heizkraftwerk und Vergärungsanlage mittels einer Leitung miteinander verbunden sind, um Wärme des Heizkraftwerkes für die Vergärungsanlage zu nutzen. Dafür wird ein Teil des in der Vergärungsanlage produzierten Methangases in einem Blockheizkraftwerk verbrannt, um Wärme für die Biogasanlage und Strom für ein Stromnetz zu erzeugen. Derartige Anlagen sind sehr vorteilhaft, da vor allem Abwärme mit einem niedrigen Energieniveau für die Nutzung in einer Vergärungsanlage besonders geeignet ist.

Der Erfindung liegt die Aufgabe zugrunde, ein derartiges Energieerzeugungssystem weiterzuentwickeln. Diese Aufgabe wird mit einem Energieerzeugungssystem mit den Merkmalen des Patentanspruchs 1 gelöst. Ein Verfahren zur Energieerzeugung mit den Merkmalen des Anspruchs 14 zeigt eine vorteilhafte Verfahrensführung, um die entstehende Energie besonders gut zu nutzen.

Vorteilhafte Weiterbildungen sind Gegenstand der Unteransprüche.

Erfindungsgemäß sind das Heizkraftwerk und die Vergärungsanlage mittels einer Leitung miteinander verbunden, um Wärme des Heizkraftwerks für die Vergärungsanlage zu nutzen und die Elektrolyseanlage und das Heizkraftwerk stehen über eine Leitung für Gas miteinander in Verbindung, um bei der Elektrolyse entstehenden Sauerstoff für das Heizkraftwerk zu nutzen. Dies ermöglicht es, den Wirkungsgrad des Energieerzeugungssystems zu verbessern. Da die Elektrolyseanlage und das Heizkraftwerk über eine Leitung für Gas miteinander in Verbindung stehen, kann bei der Elektrolyse entstehender Sauerstoff für das Heizkraftwerk genutzt werden.

Wenn das Heizkraftwerk eine Müllverbrennungsanlage ist, kann ein Teil des Mülls in der Vergärungsanlage behandelt werden, während ein anderer Teil verbrannt wird.

In der Praxis hat sich herausgestellt, dass es vorteilhaft ist, wenn die Vergärungsanlage eine Anlage zur Trockenvergärung ist.

Eine vorteilhafte Ausgestaltung sieht vor, dass das Heizkraftwerk und die Vergärungsanlage auch über eine Stromleitung direkt miteinander verbunden sind, um am Heizkraftwerk erzeugten Strom für die Vergärungsanlage zu verwenden. Das heißt, dass ein Teil des mit dem Heizkörper erzeugten Stroms nicht in ein allgemeines Stromnetz eingespeist wird, sondern direkt für den Betrieb der Vergärungsanlage genutzt wird.

Besonders vorteilhaft ist es, wenn die Elektrolyseanlage und die Vergärungsanlage über eine Leitung für Wärme miteinander in Verbindung stehen, um die bei der Elektrolyse entstehende Wärme für die Vergärungsanlage zu nutzen.

Außerdem können die Elektrolyseanlage und die Vergärungsanlage über eine Leitung für Gas miteinander in Verbindung stehen, um bei der Elektrolyse entstehenden Wasserstoff für die Vergärungsanlage zu nutzen. Dabei kann der Wasserstoff bei der Vergärung dazu genutzt werden, die Methanerzeugung zu steigern, in dem bereits während der Vergärung Wasserstoff zugeführt wird.

Alternativ oder Kumulativ kann vorgesehen sein, dass die Elektrolyseanlage und die Vergärungsanlage über eine Leitung für Gas miteinander in Verbindung stehen, um bei der Elektrolyse entstehenden Wasserstoff mit bei der Vergärung entstehendem Gas zu mischen. Dadurch wird eine Gasveredelung erreicht.

Am Heizkraftwerk entstehender Strom kann nicht nur für die Vergärung, sondern auch für die Elektrolyse verwendet werden. Daher wird vorgeschlagen, dass die Elektrolyseanlage und das Heizkraftwerk über eine Stromleitung direkt miteinander verbunden sind, um am Heizkraftwerk erzeugten Strom für die Elektrolyseanlage zu verwenden. Der Strom wird somit nicht zunächst in ein allgemeines Stromnetz eingeleitet um daraus für die Elektrolyse verwendet zu werden, sondern eine direkte Leitung zwischen Heizkraftwerk und Elektrolyseanlage ermöglicht die Nutzung von am Heizkraftwerk erzeugtem Strom für die Elektrolyse.

Entsprechend können die Elektrolyseanlage und das Heizkraftwerk auch über eine Leitung miteinander verbunden sein, um am Heizkraftwerk erzeugten Dampf für die Elektrolyseanlage zu verwenden. Die Leitung kann somit beispielsweise eine Dampf- oder Heißwasserleitung sein.

Die einzelnen Anlagen, bei denen Gas anfällt, das in einer anderen Anlage genutzt werden soll, haben naturgemäß einen gewissen Gasspeicher, in dem das Gas anfällt. Um zwischen den Komponenten des Energieerzeugungssystems den Gasaustausch zu kontrollieren und/oder das Gas für Dritte bereitzustellen, wird vorgeschlagen, dass das Energieerzeugungssystem einen Gasspeicher aufweist, der ein Volumen von mehr als 50 m³ hat.

Eine besondere Weiterbildung des Energieerzeugungssystems sieht vor, dass es eine Methangaserzeugungsanlage aufweist, die mittels Leitungen für Kohlendioxid mit dem Heizkraftwerk und/oder der Vergärungsanlage als auch über eine Leitung für Wasserstoff mit der Elektrolyseanlage in Verbindung steht. Dies ermöglicht es, eine Methangaserzeugungsanlage besonders vorteilhaft in das Energieerzeugungssystem zu integrieren.

Vorteilhaft ist es dabei, wenn die Methangaserzeugungsanlage über eine Leitung für Wärme mit dem Heizkraftwerk und/oder mit der Elektrolyseanlage in Verbindung steht. Dadurch kann auch intern erzeugte Wärme für die Methangaserzeugungsanlage verwendet werden.

Entsprechend kann auch intern erzeugter Strom für die Methangaserzeugungsanlage verwendet werden, wenn die Methangaserzeugungsanlage über eine Stromleitung direkt mit dem Heizkraftwerk in Verbindung steht, um am Heizkraftwerk erzeugten Strom für die Methangaserzeugungsanlage zu verwenden.

Entsprechend sieht ein Verfahren zur Energieerzeugung mit einem Energieerzeugungssystem nach einem der Ansprüche 1-13 vor, Wärme eines Heizkraftwerkes für eine Vergärungsanlage zu verwenden und zusätzlich Wärme einer Elektrolyseanlage für die Vergärungsanlage zu verwenden, wobei der Sauerstoff der Elektrolyse für das Heizkraftwerk verwendet wird.

Dabei ist es vorteilhaft, wenn Wasserstoff der Elektrolyseanlage mit Kohlendioxid der Trockenvergärung zu Methan umgewandelt wird.

Um bei dem Verfahren produziertes Gas kontrolliert einer Weiterverwendung zuzuführen, wird vorgeschlagen, dass mindestens ein im Energieerzeugungssystem entstehender Gasstrom gespeichert wird. Dieser Gasstrom kann Kohlendioxid, Wasserstoff, Sauerstoff, Methan und/oder Biogas sein. Dies ermöglicht es, die Gasproduktion und den Gasverbrauch in den einzelnen Komponenten der Anlage leichter zu steuern, da die Speicher zumindest in begrenztem Umfang eine zeitliche Kopplung ermöglichen.

Vorteilhafte Ausführungsbeispiele sind in der Zeichnung dargestellt und werden im Folgenden näher erläutert. Es zeigt:
- Figur 1: ein Energieerzeugungssystem mit Heizkraftwerk, Vergärungsanlage und Elektrolyseanlage,
- Figur 2: ein Energieerzeugungssystem gemäß Figur 1 mit einer Methangaserzeugungsanlage und
- Figur 3: ein Energieerzeugungssystem gemäß Figur 2 mit Gasspeichern.

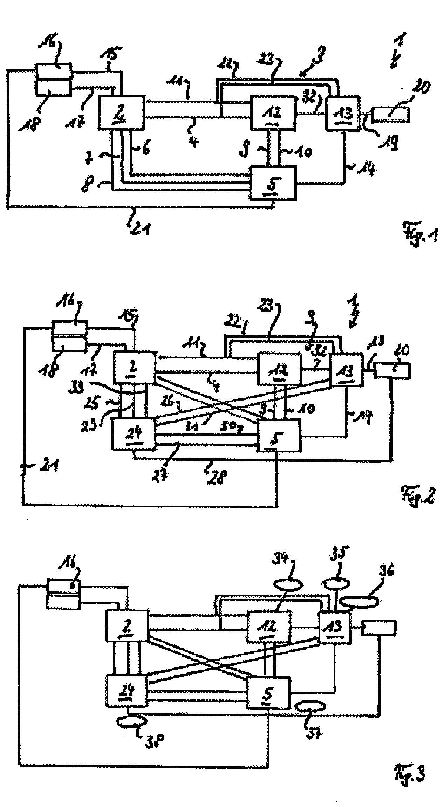

Das in Figur 1 erzeugte Energieerzeugungssystem 1 weist als Heizkraftwerk 2 eine Müllverbrennungsanlage auf. Diese Müllverbrennungsanlage liefert Energie für eine Vergärungsanlage 3, die als Trockenvergärungsanlage ausgebildet ist. Hierfür ist eine Leitung 4 zwischen dem Heizkraftwerk 2 und der Vergärungsanlage 3 angeordnet, damit Wärme des Heizkraftwerks 2 für die Vergärungsanlage 3 genutzt werden kann.

Darüber hinaus weist das Energieerzeugungssystem 1 eine Elektrolyseanlage 5 auf, die über Leitungen 6 bis 10 sowohl mit dem Heizkraftwerk 2 als auch mit der Vergärungsanlage 3 in Verbindung steht.

Eine Stromleitung 11 verbindet das Heizkraftwerk 2 direkt mit der Vergärungsanlage 3, um am Heizkraftwerk 2 erzeugten Strom für die Vergärungsanlage 3 zu verwenden.

Die Elektrolyseanlage 5 und die Vergärungsanlage 3 stehen über einer Leitung 9 für Wärme und über einer Leitung 10 für Gas miteinander in Verbindung, um bei der Elektrolyse entstehende Wärme und bei der Elektrolyse entstehenden Wasserstoff für die Vergärungsanlage 3 zu nutzen. Die Vergärungsanlage 3 weist eine Anlage zur Trockenvergärung 12 und eine Gasveredlungsanlage 13 auf. Dies ermöglicht es, über eine Leitung 14 zwischen der Elektrolyseanlage 5 und der Vergärungsanlage 3 bei der Elektrolyse entstehenden Wasserstoff in der Gasveredlungsanlage 13 mit in der Vergärungsanlage 3 zu mischen.

Die Elektrolyseanlage und das Heizkraftwerk 2 stehen über einer Leitung 6 miteinander in Verbindung und um am Heizkraftwerk 2 erzeugten Dampf oder Wärme für die Elektrolyseanlage 5 zu verwenden. Eine Stromleitung 7 zwischen Elektrolyseanlage 5 und Heizkraftwerk 2 ermöglicht es, am Heizkraftwerk 2 erzeugten Strom direkt für die Elektrolyseanlage zu verwenden und eine Leitung 8 für Gas zwischen Elektrolyseanlage 5 und dem Heizkraftwerk 2 ermöglicht es, bei der Elektrolyse entstehenden Sauerstoff für das Heizkraftwerk 2 zu nutzen.

Dieses Energieerzeugungssystem 1 ist über eine Stromleitung 15 mit einem Stromnetz 16 verbunden. Eine Leitung 17 bindet das Energieerzeugungssystem 1 an ein Wärmenetz 18 an und eine Leitung 19 verbindet die Vergärungsanlage 3 mit einer Gasversorgung 20. Dies ermöglicht es, über die Leitung 15 Strom, über die Leitung 17 Wärme und über die Leitung 19 Gas aus dem Energieerzeugungssystem abzuführen.

Eine Leitung 21 ermöglicht die zusätzliche Versorgung der Elektrolyseanlage 5 mit Strom aus dem Stromnetz 16. Außerdem kann die Gasveredelungsanlage 13 über eine Stromleitung 22 mit Strom des Heizkraftwerks 2 und über einer Leitung 23 mit Wärme des Heizkraftwerks 2 versorgt werden.

Die Figur 2 zeigt die Einbindung einer Methangaserzeugungsanlage 24 in das in Figur 1 gezeigte Energieerzeugungssystem 1. Dabei sind gleiche Komponenten mit gleichen Bezugsziffern versehen. Die Methangaserzeugungsanlage 24 ist als P2G (Power-to-Gas-Anlage) ausgebildet und erhält über die Leitung 25 CO₂ vom Heizkraftwerk 2 und über die Leitung 26 CO₂ aus der Gasveredelungsanlage 13 der Vergärungsanlage 3. Mit über die Leitung 27 von der Elektrolyseanlage 5 zugeführtem Wasserstoff, erzeugt die Methangaserzeugungsanlage Methan, das über die Leitung 28 der Gasversorgung 20 zugeführt wird. Eine Leitung 29 versorgt die Methangaserzeugungsanlage 24 mit Wärme aus dem Heizkraftwerk 2 und eine Leitung 30 versorgt die Methangaserzeugungsanlage 24 mit Wärme aus der Elektrolyseanlage 5.

Das in der Methangaserzeugungsanlage 24 entstehende Gas kann entweder über die Leitung 28 direkt der Gasversorgung zugeführt werden oder über eine Leitung 31 zunächst der Gasveredelungsanlage 13 zugeführt werden. Diese Gasveredelungsanlage 13 erhält von der Trockenvergärungsanlage 12 mit integrierter Gasaufbereitung über die Leitung 32 Biomethangas.

Die Leitung 33 dient als Stromleitung der Versorgung der Methangaserzeugungsanlage 24 mit Strom aus dem Heizkraftwerk 2.

Die Kombination von Heizkraftwerk 2, Trockenvergärungsanlage 12 und Elektrolyseanlage 5 ermöglicht es, die Trockenvergärungsanlage 12 und die Elektrolyseanlage 5 mit Energie vom Heizkraftwerk zu versorgen. Die Elektrolyseanlage 5 verbessert dadurch den Wirkungsgrad des Heizkraftwerks 2 und der Trockenvergärungsanlage 12 und sie erhöht den Heizwert des in der Vergärungsanlage 3 erzeugten Gases. Außerdem entkoppelt die Elektrolyse als Stromsenke den Kreislauf vom Stromnetz.

Die weitere Einbindung der Methangaserzeugungsanlage 24 nutzt diese Anlage als CO₂ Senke und ermöglicht eine Nutzung des bei der Trockenvergärungsanlage 12 anfallenden Biogenen CO₂ (Carbon Capture, negative CO₂ Bilanz). Außerdem ermöglicht die Methangaserzeugungsanlage als Wasserstoffsenke eine weitere Entkopplung vom Stromnetz.

Die Figur 3 zeigt, wie die Trockenvergärungsanlage 12 mit einem Speicher 34 für Biogas und die Gasveredelungsanlage 13 mit Speichern 35 für CO₂ und 36 für Biomethangas verbunden ist. Die Elektrolyseanlage 5 ist mit einem Speicher 37 für Wasserstoff und die Methangaserzeugungsanlage 24 ist mit einem Speicher 38 für Methangas verbunden. Diese Speicher ermöglichen vor allem eine verstärkte Entkopplung vom Stromnetz 16.

## Patentansprüche

1. Energieerzeugungssystem (1) mit einem Heizkraftwerk (2), einer Vergärungsanlage (3), und einer Elektrolyseanlage (5), die über eine Leitung (10) mit der Vergärungsanlage (3) in Verbindung steht, , ***dadurch gekennzeichnet, dass*** das Heizkraftwerk (2) und die Vergärungsanlage (3) mittels einer Leitung (4) miteinander verbunden sind, um Wärme des Heizkraftwerks (2) für die Vergärungsanlage (3) zu nutzen und die Elektrolyseanlage (5) und das Heizkraftwerk (2) über eine Leitung (8) für Gas miteinander in Verbindung stehen, um bei der Elektrolyse entstehenden Sauerstoff für das Heizkraftwerk (2) zu nutzen.

2. Energieerzeugungssystem nach Anspruch 1, ***dadurch gekennzeichnet, dass*** das Heizkraftwerk (2) eine Müllverbrennungsanlage ist.

3. Energieerzeugungssystem nach einem der vorhergehenden Ansprüche, ***dadurch gekennzeichnet, dass*** die Vergärungsanlage (3) eine Anlage zur Trockenvergärung (12) aufweist.

4. Energieerzeugungssystem nach einem der vorhergehenden Ansprüche, ***dadurch gekennzeichnet, dass*** das Heizkraftwerk (2) und die Vergärungsanlage (3) über eine Stromleitung (11) direkt miteinander verbunden sind, um am Heizkraftwerk (2) erzeugten Strom für die Vergärungsanlage (3) zu verwenden.

5. Energieerzeugungssystem nach einem der vorhergehenden Ansprüche, ***dadurch gekennzeichnet, dass*** die Elektrolyseanlage (5) und die Vergärungsanlage (3) über eine Leitung (9) für Wärme miteinander in Verbindung stehen, um bei der Elektrolyse entstehende Wärme für die Vergärungsanlage (3) zu nutzen.

6. Energieerzeugungssystem nach einem der vorhergehenden Ansprüche, ***dadurch gekennzeichnet, dass*** die Elektrolyseanlage (5) und die Vergärungsanlage (3) über eine Leitung (10) für Gas miteinander in Verbindung stehen, um bei der Elektrolyse entstehenden Wasserstoff für die Vergärungsanlage (3) zu nutzen.

7. Energieerzeugungssystem nach einem der vorhergehen Ansprüche, ***dadurch gekennzeichnet, dass*** die Elektrolyseanlage (5) und die Vergärungsanlage (3) über eine Leitung (14) für Gas miteinander in Verbindung stehen, um bei der Elektrolyse entstehenden Wasserstoff in einer Gasveredelungsanlage (13) mit in der Vergärungsanlage (3) entstehendem Gas zu mischen.

8. Energieerzeugungssystem nach einem der vorhergehenden Ansprüche, ***dadurch gekennzeichnet, dass*** die Elektrolyseanlage (5) und das Heizkraftwerk (2) über eine Stromleitung (7) direkt miteinander verbunden sind, um am Heizkraftwerk (2) erzeugten Strom für die Elektrolyseanlage (5) zu verwenden.

9. Energieerzeugungssystem nach einem der vorhergehenden Ansprüche, ***dadurch gekennzeichnet, dass*** die Elektrolyseanlage (5) und das Heizkraftwerk (2) über eine Leitung (6) miteinander verbunden sind, um am Heizkraftwerk (2) erzeugten Dampf für die Elektrolyseanlage (5) zu verwenden.

10. Energieerzeugungssystem nach einem der vorhergehenden Ansprüche, ***dadurch gekennzeichnet, dass*** es mindestens einem Gasspeicher (34, 35, 36, 37, 38) aufweist, der ein Volumen von mehr als 50 m³ hat.

11. Energieerzeugungssystem nach einem der vorhergehenden Ansprüche, ***dadurch gekennzeichnet, dass*** das Energieerzeugungssystem (1) eine Methangaserzeugungsanlage (24) aufweist, die mittels Leitungen (25, 26) für Kohlendioxid mit dem Heizkraftwerk (2) und/oder der Vergärungsanlage (3) als auch über eine Leitung (27) für Wasserstoff mit der Elektrolyseanlage (5) in Verbindung steht.

12. Energieerzeugungssystem nach Anspruch 11, ***dadurch gekennzeichnet, dass*** die Methangaserzeugungsanlage (24) über eine Leitung (29) für Wärme mit dem Heizkraftwerk (2) und/oder mit der Elektrolyseanlage (5) in Verbindung steht.

13. Energieerzeugungssystem nach Anspruch 11 oder 12, ***dadurch gekennzeichnet, dass*** die Methangaserzeugungsanlage (24) über eine Stromleitung (33) direkt mit dem Heizkraftwerk (2) in Verbindung steht, um am Heizkraftwerk (2) erzeugten Strom für die Methangaserzeugungsanlage (24) zu verwenden.

14. Verfahren zur Energieerzeugung mit einem System nach einem der vorhergehenden Ansprüche, bei dem Wärme eines Heizkraftwerks (2) für eine Vergärungsanlage (3) verwendet wird, ***dadurch gekennzeichnet, dass*** zusätzlich Wärme einer Elektrolyseanlage (5) für die Vergärungsanlage (3) verwendet wird und der Sauerstoff der Elektrolyseanlage (5) für das Heizkraftwerk (2) verwendet wird.

15. Verfahren nach Anspruch 14, ***dadurch gekennzeichnet, dass*** am Heizkraftwerk (2) erzeugter Dampf für die Elektrolyseanlage (5) verwendet wird.

16. Verfahren nach Anspruch 14 oder 15, ***dadurch gekennzeichnet, dass*** Wasserstoff der Elektrolyseanlage (5) mit Kohlendioxid der Vergärung zu Methan umgewandelt wird.

17. Verfahren nach einem der Ansprüche 14 bis 16, ***dadurch gekennzeichnet, dass*** mindestens ein im Energieerzeugungssystem (1) entstehender Gasstrom gespeichert wird.

18. Verfahren nach einem der Ansprüche 14 bis 17, ***dadurch gekennzeichnet, dass*** produziertes und gespeichertes Gas kontrolliert einer Weiterverwendung zugeführt wird.

## Claims

1. Energy generating system (1) with a cogeneration plant (2), a fermentation plant (3), and an electrolysis system (5) which is connected to the fermentation plant (3) via a line (10) ***characterized in that*** the cogeneration plant (2) and the fermentation plant (3) are connected to one another by means of a line (4) in order to use heat from the cogeneration plant (2) for the fermentation plant (3), and the electrolysis system (5) and the cogeneration plant (2) are connected to one another via a line (8) for gas in order to use oxygen generated during the electrolysis for the cogeneration plant (2).

2. Energy generating system according to Claim 1, ***characterized in that*** the cogeneration plant (2) is a waste incineration plant.

3. Energy generating system according to any one of the preceding claims, ***characterized in that*** the fermentation plant (3) includes a plant for dry fermentation (12).

4. Energy generating system according to any one of the preceding claims, ***characterized in that*** the cogeneration plant (2) and the fermentation plant (3) are connected directly to one another via a power supply line (11) so that electrical current generated in the cogeneration plant (2) can be used for the fermentation plant (3).

5. Energy generating system according to any one of the preceding claims, ***characterized in that*** the electrolysis system (5) and the fermentation plant (3) are connected to one another via a line (9) for heat, so that heat generated during the electrolysis can be used for the fermentation plant (3).

6. Energy generating system according to any one of the preceding claims, ***characterized in that*** the electrolysis system (5) and the fermentation plant (3) are connected to one another via a line (10) for gas in order to use hydrogen generated during the electrolysis for the fermentation plant (3).

7. Energy generating system according to any one of the preceding claims, ***characterized in that*** the electrolysis system (5) and the fermentation plant (3) are connected to one another via a line (14) for gas in order to mix hydrogen generated during the electrolysis with gas generated in the fermentation plant (3) in a gas refining plant (13).

8. Energy generating system according to any one of the preceding claims, ***characterized in that*** the electrolysis system (5) and the cogeneration plant (2) are connected directly to one another via a power supply line (7) so that the electrical power generated in the cogeneration plant (2) can be used for the electrolysis system (5).

9. Energy generating system according to any one of the preceding claims, ***characterized in that*** the electrolysis system (5) and the cogeneration plant (2) are connected to one another via a line (6) so that the steam generated in the cogeneration plant (2) can be used for the electrolysis system (5).

10. Energy generating system according to any one of the preceding claims, ***characterized in that*** it includes at least one gas storage tank (34, 35, 36, 37, 38) with a volume of more than 50 m³.

11. Energy generating system according to any one of the preceding claims, ***characterized in that*** the energy generating system (1) includes a methane gas generation plant (24) which is connected to the cogeneration plant (2) and/or the fermentation plant (3) via lines (25, 26) for carbon dioxide and connected to the electrolysis system (5) via a line (27) for hydrogen.

12. Energy generating system according to Claim 11, ***characterized in that*** the methane gas generation plant (24) is connected to the cogeneration plant (2) and/or the electrolysis system (5) via a line (29) for heat.

13. Energy generating system according to Claim 11 or 12, ***characterized in that*** the methane gas generation plant (24) is connected directly to the cogeneration plant (2) via a power supply line (33) so that the electrical power generated in the cogeneration plant (2) can be used for the methane gas generation plant (24).

14. Method for the production of energy, including in particular a system according to any one of the preceding claims, in which heat from a cogeneration plant (2) is used for a fermentation plant (3), ***characterized in that*** additionally heat from an electrolysis system (5) is used for the fermentation plant (3) and the oxygen from the electrolysis system (5) is used for the cogeneration plant (2).

15. Method according to Claim 14, ***characterized in that*** steam generated in the cogeneration plant (2) is used for the electrolysis system (5).

16. Method according to Claim 14 or 15, ***characterized in that*** hydrogen from the electrolysis system (5) is converted into methane using carbon dioxide from the fermentation.

17. Method according to any one of Claims 14 to 16, ***characterized in that*** at least one gas stream generated in the energy generating system (1) is stored.

18. Method according to any one of Claims 14 to 17, ***characterized in that*** gas which has been produced and stored is transported under control for further use.

## Revendications

1. Système de production d'énergie (1) doté d'une centrale combinée (2), d'une installation de fermentation (3) et d'une installation d'électrolyse (5), qui est en liaison par le biais d'un conduit (10) avec l'installation de fermentation (3), ***caractérisé en ce que*** la centrale combinée (2) et l'installation de fermentation (3) sont reliées entre elles par le biais d'un conduit (4) pour utiliser la chaleur de la centrale combinée (2) pour l'installation de fermentation (3) et l'installation d'électrolyse (5) et la centrale combinée (2) sont en liaison entre elles pour le gaz par le biais d'un conduit (8) pour le gaz pour utiliser l'oxygène produit lors de l'électrolyse pour la centrale combinée (2).

2. Système de production d'énergie selon la revendication 1, ***caractérisé en ce que*** la centrale combinée (2) est une installation d'incinération d'ordures ménagères.

3. Système de production d'énergie selon l'une quelconque des revendications précédentes, ***caractérisé en ce que*** l'installation de fermentation (3) comporte une installation pour la fermentation à sec (12).

4. Système de production d'énergie selon l'une quelconque des revendications précédentes, ***caractérisé en ce que*** la centrale combinée (2) et l'installation de fermentation (3) sont directement reliées entre elles par le biais d'un conduit de courant (11) pour utiliser le courant produit à la centrale combinée (2) pour l'installation de fermentation (3).

5. Système de production d'énergie selon l'une quelconque des revendications précédentes, ***caractérisé en ce que*** l'installation d'électrolyse (5) et l'installation de fermentation (3) sont en liaison entre elles pour la chaleur par le biais d'un conduit (9) pour utiliser la chaleur produite lors de l'électrolyse pour l'installation de fermentation (3).

6. Système de production d'énergie selon l'une quelconque des revendications précédentes, ***caractérisé en ce que*** l'installation d'électrolyse (5) et l'installation de fermentation (3) sont en liaison entre elles par le biais d'un conduit (10) pour le gaz pour utiliser l'hydrogène produit lors de l'électrolyse pour l'installation de fermentation (3).

7. Système de production d'énergie selon l'une quelconque des revendications précédentes, ***caractérisé en ce que*** l'installation d'électrolyse (5) et l'installation de fermentation (3) sont en liaison entre elles par le biais d'un conduit (14) pour le gaz pour mélanger l'hydrogène produit lors de l'électrolyse dans une installation d'affinage de gaz (13) avec le gaz se formant dans l'installation de fermentation (3).

8. Système de production d'énergie selon l'une quelconque des revendications précédentes, ***caractérisé en ce que*** l'installation d'électrolyse (5) et la centrale combinée (2) sont reliées directement entre elles par le biais d'un conduit de courant (7) pour utiliser le courant produit à la centrale combinée (2) pour l'installation d'électrolyse (5).

9. Système de production d'énergie selon l'une quelconque des revendications précédentes, ***caractérisé en ce que*** l'installation d'électrolyse (5) et la centrale combinée (2) sont reliés entre elles par le biais d'un conduit (6) pour utiliser la vapeur produite à la centrale combinée (2) pour l'installation d'électrolyse (5).

10. Système de production d'énergie selon l'une quelconque des revendications précédentes, ***caractérisé en ce qu'***il comporte au moins un accumulateur de gaz (34, 35, 36, 37, 38), qui a un volume de plus de 50 m³.

11. Système de production d'énergie selon l'une quelconque des revendications précédentes, ***caractérisé en ce que*** le système de production d'énergie (1) comporte une installation de production de gaz de méthane (24), qui est en liaison au moyen de conduits (25, 26) pour dioxyde de carbone avec la centrale combinée (2) et/ou l'installation de fermentation (3) ainsi que par le biais d'un conduit (27) pour oxygène avec l'installation d'électrolyse (5).

12. Système de production d'énergie selon la revendication 11, ***caractérisé en ce que*** l'installation de production de gaz de méthane (24) est en liaison avec la centrale combinée (2) et/ou avec l'installation d'électrolyse (5) par le biais d'un conduit (29) pour la chaleur.

13. Système de production d'énergie selon la revendication 11 ou 12, ***caractérisé en ce que*** l'installation de production de gaz de méthane (24) est en liaison par le biais d'un conduit de courant, directement avec la centrale combinée (2) par le biais d'un conduit de courant (33) pour utiliser le courant produit à la centrale combinée (2) pour l'installation de production de gaz de méthane (24).

14. Procédé destiné à la production d'énergie avec un système selon l'une quelconque des revendications précédentes, pour lequel la chaleur d'une centrale combinée (2) est utilisée pour une installation de fermentation (3), ***caractérisé en ce que*** de la chaleur d'une installation d'électrolyse (5) est utilisée en plus pour l'installation de fermentation (3) et l'oxygène de l'installation d'électrolyse (5) est utilisé pour la centrale combinée (2).

15. Procédé selon la revendication 14, ***caractérisé en ce que*** la vapeur produite à la centrale combinée (2) est utilisée pour l'installation d'électrolyse (5).

16. Procédé selon la revendication 14 ou 15, ***caractérisé en ce que*** l'hydrogène de l'installation d'électrolyse (5) est transformé en méthane avec du dioxyde de carbone de la fermentation.

17. Procédé selon l'une quelconque des revendications 14 à 16, ***caractérisé en ce qu'***au moins un flux de gaz se produisant dans le système de production d'énergie (1) est stocké.

18. Procédé selon l'une quelconque des revendications 14 à 17, ***caractérisé en ce que*** le gaz produit et accumulé est dirigé de façon contrôlée vers une réutilisation.
